# EUROPEAN PATENT APPLICATION

(11) **EP 4 316 498 A1**
(43) Date of publication of application: **07.02.2024**
(21) Application number: 22780725.2
(22) Date of filing: 28.03.2022
(51) Int. Cl.: A61K 35/50, A61K 35/28, A61P 43/00, C12N 5/0775

(54) **TRYPSIN INHIBITION METHOD, AND METHOD FOR PRODUCING CELL PREPARATION IN WHICH SAME IS USED**

(30) Priority: 30.03.2021 JP 2021056659
(71) Applicant: Kaneka Corporation, Osaka-shi, Osaka 530-8288 (JP)
(72) Inventor: NAKAISHI, Tomoyuki, Kobe-shi, Hyogo 650-0047 (JP); NAKAYAMA, Soya, Kobe-shi, Hyogo 650-0047 (JP); IKEBUCHI, Fumie, Kobe-shi, Hyogo 650-0047 (JP); MIYOSHI NAKASHIMA, Sayaka, Kobe-shi, Hyogo 650-0047 (JP)
(74) Representative: Hoffmann Eitle
(86) International application number: PCT/JP2022/014927
(87) International publication number: WO 2022/210514

(57) **Abstract**

There was no method for producing a cell preparation which contains an amount of xenogenic component as small as possible and is highly safe in order to reduce the xenogenic rejection and the risk of transmission of a prion or a viral pathogen after trypsin was used for releasing and dispersing cells. Provided is a method for producing a cell preparation, comprising: a step of culturing a cell which adheres to a base material, a step of releasing the cell from the base material using trypsin, and a step of contacting a human platelet lysate (hPL) with both the trypsin and the cell after said releasing.

## Description

### Technical Field

The present invention relates to a method for inhibiting trypsin which is used to release and disperse cells, and a method for producing a cell preparation using the same.

### Background Art

Cell culture in vitro includes suspension culture and adherent culture. In adherent culture, cells proliferate while adhering to a base material. When cells have grown all over the base material, leaving no more space (becoming confluent), the cells can no longer grow, and thus, at this stage or immediately before the stage, the cells are released from the base material, collected, and stored, and for further proliferation, the cells collected are diluted and then seeded on a new base material and cultured again. In such a case, the cells are released most commonly using trypsin or trypsin-EDTA.

A common cell releasing method is performed as follows: a culture medium is removed from a base material to which cells adhere; then the cells are washed with, for example, an aqueous solution comprising neither calcium nor magnesium (for example, a PBS (-) solution or a physiological saline); then the aqueous solution is removed; a releasing agent such as trypsin or trypsin-EDTA solution is added to the cells on the base material until the cells are completely immersed; the cells are left to stand at 37°C for approximately 2 to 10 minutes; and it is confirmed that the cells are released from the base material. If the trypsin activity remains still after the cells are released, the cells can be damaged, and thus, the releasing treatment needs to be followed by inactivating the trypsin. Trypsin is inactivated commonly as follows: trypsin is diluted with an aqueous solution added, or washing is performed using centrifugation or the like; or a solution mixture after trypsin reaction is contacted with a trypsin inhibitor such as those soybean-derived or with an FBS (fetal bovine serum) or serum-comprising culture medium known to have a trypsin-inhibiting activity, so that the remaining trypsin is inactivated, and then, the cells are collected by centrifugation and washed with a suitable salt solution or a culture medium; and then, the cells are stored, or further cultured with a suitable culture medium.

Patent Literature 1 describes trypsin treatment of cells in the production of therapeutic cells such as mesenchymal stem cells. Specifically, cells are released by trypsin treatment, washed with a Hank's basic salt solution (HBSS) + 1% recombinant human serum albumin (HSA), then re-suspended at a concentration of 10⁷ cells/ml in an HBSS + 1% HSA, fucosylated, washed, and then cryopreserved in the presence of a cryopreservation agent and a physiological balanced salt solution comprising a human platelet lysate and the like.

Patent Literature 2 describes a method for producing cells that secrete a neurotrophic factor (NTF), in which subculture of undifferentiated mesenchymal stem cells include trypsin dilution treatment with a suitable culture medium, for example, a Hank's balanced salt solution (HBSS), Dulbecco's modified Eagle's medium (DMEM), RPMI, PBS, or the like after trypsin treatment, followed by collecting the cells.

Patent Literature 3 discloses that adhering cells are treated with trypsin, washed with an αMEM culture medium, and cryopreserved with 10% DMSO, 5% human serum albumin, and PlasmaLyte.

### Citation List

### Patent Literature

Patent Literature 1: JP 2017-525340 A
Patent Literature 2: JP 2015-531594 A
Patent Literature 3: JP 2011-529706 A

### Non-Patent Literature

Non-Patent Literature 1: Eastment CT et al., J Cell Physiol. 1978 Oct; 97(1):17-27
Non-Patent Literature 2: Schallmoser k et al., Methods Mol Biol. 2013; 946:349-62

### Summary of Invention

### Technical Problem

As described above, trypsin is commonly inactivated by contacting a solution mixture of trypsin reaction with a trypsin inhibitor such as those derived from soybean, or an FBS (fetal bovine serum) or serum-comprising culture medium having a trypsin-inhibiting activity. However, an FBS and a soybean-derived trypsin inhibitor are xenogenic components for humans, involve the risk of xenogenic rejection, and thus, and are considered to be not necessarily suitable for use in the production of a cell preparation intended to be administered to a human. In addition, use of an FBS includes the risk of transmission of a prion or a viral pathogen. Accordingly, in the production of a cell preparation to be actually administered to a human, it is preferable from a safety viewpoint to use a trypsin inhibitor which does not comprise a xenogenic component as a main component.

A problem to be solved by the present invention is to provide an agent for inactivating trypsin which does not comprise a xenogenic component as a main component, to find a method for efficiently inactivating trypsin using said agent for inactivating trypsin after treating cells with trypsin, and to provide a highly safe cell preparation which involves use of xenogenic component in an amount as small as possible in the production process.

### Solution to Problem

The present inventors have intensively studied to solve the above-described problems, and as a result, have considered that a human albumin formulation and a human platelet lysate (hPL) can be a candidate for a trypsin inhibitor which does not comprise a xenogenic component as a main component. An hPL is produced, for example, from a blood platelet formulation for transfusion, and can be used as an additive for a basal medium in human cell culture. A product for production of cells for clinical use is sold, and can be utilized for production of a cell preparation as well as a human albumin formulation.

There is no report that an hPL was utilized as a trypsin inhibitor, but as a result of the studies, the present inventors have found that an hPL has a strong activity of inhibiting trypsin, and is very effective as an agent for inactivating trypsin. In addition, the present inventors have found an appropriate amount of the agent for inactivating trypsin to be used with respect to the amount of trypsin, have found that a particular treatment is preferably carried out for an hPL before use, and have established a method for producing a highly safe cell preparation.

That is, the present invention provides the following invention.
(1) A method for producing a cell preparation, comprising a step of culturing a cell which adheres to a base material, a step of releasing the cell from the base material using trypsin, and a step of contacting a human platelet lysate (hPL) with both the trypsin and the cell after said releasing.
(2) The method for producing a cell preparation of (1), wherein the hPL is clarified before contacting with the trypsin.
(3) The method for producing a cell preparation of (1) or (2), wherein the method uses an undiluted hPL.
(4) The method for producing a cell preparation of any of (1) to (3), wherein the amount of the contacted hPL is 1.44 µL or more per µg of the trypsin.
(5) The method for producing a cell preparation of (4), wherein the amount of the contacted hPL is 2.4 µL or more per µg of the trypsin.
(6) The method for producing a cell preparation of (5), wherein the cell is treated with trypsin in the concentration of is 0.9 µg/mL or more and 83.3 µg/mL or less in the step of releasing the cell from the base material using trypsin.
(7) The method for producing a cell preparation of (6), wherein the trypsin treatment is performed at 10°C or higher and 37°C or lower in the step of releasing the cell from the base material using trypsin.
(8) The method for producing a cell preparation of any of (1) to (7), wherein the cell is cultured using a culture liquid comprising an hPL in the step of culturing a cell which adheres to a base material.
(9) The method for producing a cell preparation of any of (1) to (8), wherein the cell expresses a platelet-derived growth factor receptor (PDGFR).
(10) The method for producing a cell preparation of any of (1) to (9), wherein the cell is a mesenchymal stem cell (MSC).
(11) The method for producing a cell preparation according to (10), wherein the MSC is derived from an amnion.
(12) A cell preparation produced using a method for producing a cell preparation, the method comprising: a step of culturing a cell which adheres to a base material, a step of releasing the cell from the base material using trypsin, and a step of contacting 1.44 µL or more of an hPL per µg of the trypsin with both the trypsin and the cell after said releasing.

The present specification encompasses the disclosure of Japanese Patent Application No. 2021-056659 which forms the basis of the priority of the present application.

### Advantageous Effects of Invention

The production method of the present invention reduces the xenogenic rejection and the risk of transmission of a prion or a viral pathogen, and thus allows for production of a highly safe cell preparation comprising a xenogenic component in an amount as small as possible.

### Brief Description of Drawing

[Figure 1] Figure 1 is related to a clarification method and absorbance with respect to Example 1 in the present invention.
[Figure 2] Figure 2 is related to the amount of added hPL and the remaining trypsin activity with respect to Example 2 in the present invention.
[Figure 3] Figure 3 is related to the concentration of trypsin and the ration of cell collection with respect to Example 3 in the present invention.
[Figure 4] Figure 4 is related to the amount of added hPL and the remaining trypsin activity with respect to Example 4 in the present invention.

### Description of Embodiments

Embodiments of the present invention are described below. The production method of the present invention comprises, as essential steps, a step of culturing a cell which adheres to a base material, a step of releasing the cell from the base material using trypsin, and a step of contacting an hPL with both the trypsin and the cell after said releasing.

A base material to be used in the present invention is a solid or semisolid to which a cell is allowed to adhere. Examples of the material comprise, but are not limited to, glass, plastic (for example, a plastic the surface of which is hydrophilized), cellulose, synthesized resin, natural resin, metal, and the like. In some of the cases where the culture is performed using a cell spheroid, the cells themselves may correspond to a base material. Examples of the shape comprise a triangular prism, cube, polygonal column such as a rectangular parallelepiped, semispherical shape, and a cylinder (for example, those having a circular bottom face), or the like, and the bottom face is preferably planar (for example, rectangular or circular). Examples comprise, but are not limited to, a culture container such as a dish, petri dish, plate (for example, a multi-well plate), microcarrier, fiber, reactor, culture flask, chip, and tray. The surface of the base material such as a culture container or the like may be coated with a coating agent or the like.

Trypsin is a kind of digestive enzyme comprised in pancreatic juice, and classified as a serine protease. Trypsin has an activity of hydrolyzing a peptide bond on the carboxy group side of a basic amino acid residue such as a lysine residue or an arginine residue in a polypeptide chain. Herein, a trypsin is not particularly limited as long as it has a protease activity which a natural trypsin has. Examples include a full-length trypsin and an active fragment thereof and a modified trypsin derived from a natural trypsin in which the amino acid sequence thereof is modified. A trypsin to be used in the present invention can be used regardless of the amino acid sequence and the animal species of origin. As the amino acid sequence of trypsin, for example, the sequence from a human or a pig and a derivative thereof are well known, and such a sequence may be used, or any other sequence can be used. The origin is preferably, but not limited to, a recombinant trypsin because the present invention preferably comprises a raw material derived from an animal other than a human in an amount as small as possible. In the step of releasing a cell from the base material using trypsin, the concentration of the trypsin is not limited, and is preferably 0.01 µg/mL or more, more preferably 0.9 µg/mL or more, still more preferably 1.0 µg/mL or more, still more preferably 2.0 µg/mL or more, and preferably 500 µg/mL or less, more preferably 83.3 µg/mL or less, still more preferably 10.0 µg/mL or less, still more preferably 4.0 µg/mL or less. When the cells are released from the base material using trypsin, the trypsin, if used at a too high concentration, can adversely affect the cells, and thus, the concentration of the trypsin is preferably a low concentration in a range that the effect is produced. In addition, in the step of releasing a cell from the base material using trypsin, the reaction temperature is not limited, and preferably 4°C or higher and 40°C or lower, preferably 10°C or higher and 37°C or lower, more preferably 15°C or higher and 25°C or lower, still more preferably 18°C or higher and 23°C or lower.

An hPL means a human platelet lysate (hereinafter abbreviated as an hPL). Since an hPL comprises a growth factor and a cytokine which are useful for cell growth, an hPL is utilized as an additive for a basal medium in human cell culture. The aforementioned Non-Patent Literatures 1 and 2 exemplify a typical preparing method, without limitations thereto. An hPL to be used in the present invention preferably undergoes inactivation and/or sterilization of bacteria and viruses. An hPL may be a commercially available product. Examples comprise, but are not limited to, Stemulate (manufactured by Cook Regentec Llc), PLTMax (manufactured by Mill Creek Life Sciences, Inc.), UltraGRO (manufactured by AventaCell BioMedial Corp.), PLUS (manufactured by Compass Biomedical, Inc.), and the like.

In the present invention, an hPL to be used is preferably clarified before contacting with trypsin. Here, clarification of an hPL refers to a treatment of removing, from an hPL, an insoluble fraction generated by long-term storage or by freezing and thawing. Such an insoluble fraction is considered to have an adverse influence in a method for producing a highly safe cell preparation (for example, contamination of a cell preparation by an insoluble fraction has a risk of induction of vascular embolism when the cell preparation is administered to a human), and thus, if an insoluble fraction is present, the hPL is preferably clarified before use. However, in a case where an hPL having no insoluble fraction is used, the hPL is not necessarily clarified. In the present invention, clarification treatment is preferably performed immediately before contacting an hPL with trypsin and a cell or after thawing in a case where a cryopreserved hPL is used. Specifically, the clarification treatment is preferably performed within 7 days, within 3 days, within 2 days, within 24 hours, within 12 hours, or within 3 hours before use.

Examples of a clarification method comprise centrifugation, filtration, precipitation, and sedimentation (for example, spontaneous sedimentation), and are not limited to these as long as the method can remove an insoluble fraction. For centrifugation, conditions such as rotation speed and time are not limited, and are preferably 400 ×g for 5 minutes or longer, more preferably 1000 ×g for 5 minutes or longer, still more preferably 2000 ×g for 5 minutes or longer, still more preferably 3000 ×g for 5 minutes or longer. The filtration is not limited in terms of material or pore diameter, and the filtering medium is preferably sterilized. Preferably, the material is hydrophilic and can filtrate an hPL. The pore diameter is preferably 0.80 µm or less, still more preferably 0.45 µm or less, still more preferably 0.20 µm or less, still more preferably 0.10 µm or less. The clarification may be performed by a combination of centrifugation and filtration multiple times each, and a depth filter may be used.

The degree of clarification can be evaluated by measuring the absorbance of an hPL. For example, using, as an index, 600 nm absorbance of an insoluble fraction (turbidity) and 280 nm absorbance of an effective component (protein), an evaluation can be made with a value of 600 nm absorbance / 280 nm absorbance. A clarified hPL in the present invention means that the value of 600 nm absorbance / 280 nm absorbance is preferably 1.0 or less, more preferably 0.9 or less, more preferably 0.8 or less, still more preferably 0.7 or less, still more preferably 0.6 or less, still more preferably 0.5 or less, still more preferably 0.4 or less, still more preferably 0.3 or less, still more preferably 0.2 or less, still more preferably 0.15 or less, as measured using ultrapure water as a control liquid.

The concentration of an hPL to be contacted with both trypsin and a cell is not limited as long as an effect of inhibiting trypsin is produced. The concentration of an hPL can be calculated on the basis of the concentration of the platelet lysate protein per mL. For example, the concentration of a platelet lysate protein per mL can be 0.1 mg/mL to 500 mg/mL, 1.0 mg/mL to 400 mg/mL, 5.0 mg/mL to 300 mg/mL, 10 mg/mL to 200 mg/mL, 15 mg/mL to 100 mg/mL, 20 mg/mL to 90 mg/mL, 30 mg/mL to 80 mg/mL, 40 mg/mL to 70 mg/mL, or 50 mg/mL to 60 mg/mL, and may be, for example, 55 mg/mL.

In the present invention, an hPL to be contacted with trypsin and a cell is preferably not diluted. As used herein, an hPL which is "not diluted" specifically refers to a crude platelet lysate or a commercially available platelet lysate that is not diluted. Examples of a commercially available platelet lysate comprise Stemulate (manufactured by Cook Regentec Llc), PLTMax (manufactured by Mill Creek Life Sciences, Inc.), UltraGRO (manufactured by AventaCell BioMedial Corp.), PLUS (manufactured by Compass Biomedical, Inc.), and the like. After trypsin is inactivated with an hPL, a cell is collected by centrifugation. If an hPL diluted with a culture medium or a buffer is used, the amount of liquid and number of centrifugation undesirably increase, whereby the quality of the cell can decrease, due to increased operation time. In the industrial production of a cell preparation, such an increased operation time cannot be overlooked in some cases. Thus, from the viewpoint of not increasing the operation time, the ratio of dilution (the amount of liquid after dilution / the amount of hPL liquid before dilution) is preferably 3 times or less, more preferably 2 times or less, still more preferably 1.5 times or less, particularly preferably 1.2 times or less, in a case where a diluted hPL is used. In a case where dilution is conducted, a liquid to be used for dilution is not limited. Considering influences on a cell, the liquid preferably has an osmotic pressure in the range of 285±20 mOsm/L, more preferably in the range of 285±10 mOsm/L, more preferably in the range of 285±5 mOsm/L. Specifically, a culture medium, a physiological saline, a buffer having an osmotic pressure adjusted to the above-described range, and the like are preferable.

It is considered that the effect of inhibiting trypsin by an hPL is based on irreversible inhibition of trypsin by a component contained in the hPL which strongly binds to trypsin at a certain ratio. In the present invention, a condition for contacting an hPL with trypsin and a cell is specified in terms of the amount of hPL liquid per trypsin by weight. A plurality of products and products from different lots are available as an hPL. When effects of the above-described hPLs from a plurality of lots on the specific growth rate of an amnion-derived mesenchymal stem cell (MSC) were actually compared, it was in the range of 0.49 to 0.57 (1/day) with all of the products, and it was inferred that there is almost no difference between products and between lots. The condition of contacting is preferably 1.44 µL or more of hPL per µg of trypsin, more preferably 1.68 µL or more per µg of trypsin, still more preferably 1.92 µL or more per µg of trypsin, still more preferably 2.16 µL or more per µg of trypsin, still more preferably 2.4 µL or more, 3.2 µL or more, 3.6 µL or more, 4.2 µL or more, or 4.8 µL or more, per µg of trypsin. The upper limit is not particularly limited, and it does not technically cause problems even if a very excessive amount is used, but, for example, 100 µL or less, 75 µL or less, 50 µL or less, 30 µL or less per µg of trypsin is used, from an economical viewpoint.

In the present invention, a condition of contacting an hPL with trypsin and a cell can also be specified in terms of the amount of an hPL protein per trypsin by weight. The condition of contacting is, for example, 79.2 µg or more of hPL per µg of trypsin, 92.4 µg or more per µg of trypsin, more preferably 105.6 µg or more per µg of trypsin, more preferably 118.8 µg or more per µg of trypsin, 132 µg or more per µg of trypsin, 176 µg or more per µg of trypsin, 198 µg or more per µg of trypsin, 231 µg or more per µg of trypsin, or 264 µg or more per µg of trypsin. In addition, the condition is 5500 µg or less, 4125 µg or less, 2750 µg or less, 1650 µg or less of hPL per µg of trypsin.

The type of a cell to be used in the present invention is not particularly limited, and can be a cell that is an object to be released from a base material using trypsin after being cultured. The culture condition is not particularly limited, and the culture is preferably performed using an hPL during the culture, more specifically using a culture medium containing an hPL.

One of the active ingredients of an hPL can include a platelet-derived growth factor (PDGF), and thus, a cell as a subject in the present invention is preferably a cell which expresses a platelet-derived growth factor receptor (PDGFR). There are structurally similar two kinds of subtypes as PDGFRs, which are called PDGFR-α and -β respectively. There are dimers, i.e., PDGFR-αα, PDGFR-αβ, and PDGFR-ββ as PDGFRs, and any of them may be expressed. A derivative of a PDGFR may be expressed. In addition, the amount of the expression is not particularly limited, and adding a PDGF during culture preferably facilitates cell growth.

Examples of a cell which expresses a PDGFR include an MSC. An MSC refers to a "mesenchymal stromal cell" or a "mesenchymal stem cell", and can be utilized as a cell preparation. Examples of the origin of an MSC comprise, but are not limited to, bone marrow, hematopoietic stem cell, umbilical cord blood, umbilical cord, amnion, amniotic fluid, placental villi, nerve, adipose tissue, pancreas synovial membrane, dental pulp, deciduous tooth, sperm, testis, cornea, and the like. A cell as a subject in the present invention is preferably an MSC, and among others, is more preferably an amnion-derived MSC.

An amnion in the present invention is derived from an "appendage of fetus", and the "appendage of fetus" refers to an egg membrane, placenta, umbilical cord, and amniotic fluid. Furthermore, an "egg membrane" is a fetal sac comprising an amniotic fluid of a fetus, and consists of an amnion, chorion, and deciduum in this order from the inside. Among these, an amnion and a chorion originate from a fetus. An "amnion" refers to a transparent thin membrane which is in the innermost layer of an egg membrane, and which does not have many blood vessels. The inner layer (also referred to as an epithelial cell layer) of an amnion is covered with one layer of epithelial cells having a secretion function, and secretes an amniotic fluid. The outer layer (also referred to as an extracellular matrix layer, and corresponding to the interstitium) of an amnion comprises an MSC.

In an embodiment of the present invention, use of a human platelet lysate (hPL) as a trypsin inhibitor is provided. The use allows the inactivation of the trypsin activity which can remain in an adhering cell released from a base material using trypsin.

In a further embodiment of the present invention, a trypsin inhibitor consisting of a human platelet lysate (hPL) is provided. In addition, a composition for inhibiting trypsin comprising a trypsin inhibitor as an active ingredient is also provided. A composition for inhibiting trypsin can comprise a pharmaceutically acceptable carrier in addition to the active ingredient.

In an embodiment of the present invention, also provided is a cell preparation produced using a method for producing a cell preparation, the method comprising a step of culturing a cell which adheres to a base material, a step of releasing the cell from the base material using trypsin, and a step of contacting 1.44 µL or more of hPL per µg of trypsin (for example, 79.2 µg or more of hPL per µg of trypsin) with both the trypsin and the cell after said releasing. A cell preparation produced by the method according to the present invention comprises no xenogenic component, and the remaining activity of trypsin is reduced in the cell preparation. It is preferable that the remaining activity is not detected or that the cell preparation has no remaining activity. In a further embodiment, a cell preparation according to the present invention comprises no xenogenic component, has no remaining activity of trypsin, and can optionally comprise an hPL. A cell preparation according to the present invention produced in this way comprises substantially no xenogenic component, and thus is highly safe, and the remaining activity of trypsin is decreased, and thus, the quality of the cell is improved.

### Examples

The present invention is described in detail with reference to Examples, but the present invention is not limited to these Examples.

### (Example 1) A method for clarifying an hPL and the degree of clarification

A method for clarifying an hPL was examined. A frozen product of an hPL (having a total protein concentration of 55 mg/mL) melted at room temperature was used as a "sample after melting". A part of the "sample after melting" was centrifuged at 2000 ×g for 5 minutes, and the supernatant was used as a "centrifuged sample". A part of the "centrifuged sample" was filtrated through DISMIC-25CS (Advantec Toyo Kaisha, Ltd.) filters of 0.80 µm, 0.45 µm, and 0.20 µm, and used as a "0.80 µm sample", "0.45 µm sample", and "0.20 µm sample" respectively.

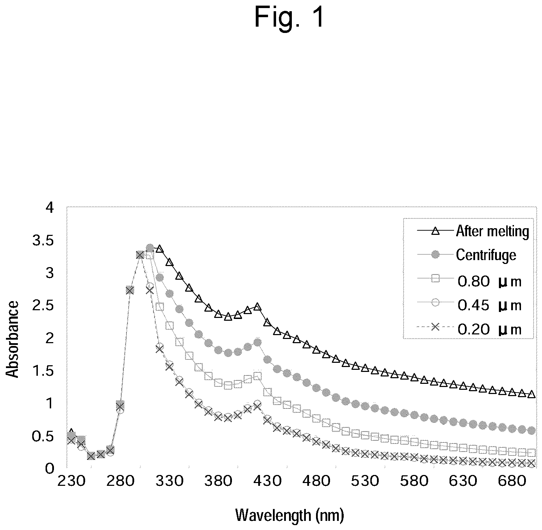

Ultrapure water was used as a control. Each of the "sample after melting", "centrifuged sample", "0.80 µm sample", "0.45 µm sample", and "0.20 µm sample" was added, 360 µL each, to a 96 Well Cell Culture Plate (REF: 3596), and the absorbance at 230 nm to 700 nm was measured (n = 3) using a microplate reader (SpectraMax i3x, Molecular Devices, LLC). Here, this measurement was performed immediately after each of the above-described samples was prepared. The area of the 96 Well Cell Culture Plate was 0.32 square centimeters, and thus, the optical path length was set at 1.125 centimeters (from 360/1000/0.32), and correction was carried out into the absorbance at 1 centimeter. From the measurement value of the sample, a difference from the value of the control was calculated to determine the absorbance. The values determined are shown in Figure 1.

Depending on the degree of centrifugation or filtration, the value of absorbance at and around 600 nm which correlates with the insoluble fraction (turbidity) decreased gradually, and on the other hand, the value of the 280 nm absorbance which correlates with the active ingredient (protein) was almost constant. Even centrifugation alone could decrease the insoluble fraction approximately to half. In the case of filtration, the insoluble fraction decreased to approximately 1/5 in the case of 0.80 µm. The results revealed that there was almost no change between 0.45 µm and 0.20 µm, and the filtration with 0.45 µm achieved substantially complete clarification. The values obtained by dividing the value of the 600 nm absorbance by the value of the 280 nm absorbance are shown in Table 1, and this also revealed that the filtration with 0.45 µm achieved almost complete clarification.

**[Table 1]**

| | After melting | Centrifuge | 0.80 µm | 0.45 µm | 0.20 µm |
|---|---|---|---|---|---|
| Absorbance 600 nm / Absorbance 280 nm | 1.364 | 0.774 | 0.365 | 0.148 | 0.149 |

### (Example 2) Inhibition of trypsin activity by addition of an hPL or HSA

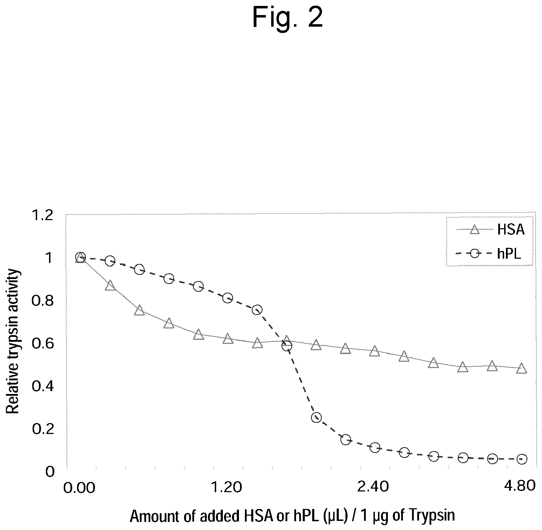

In the present Example and the below-described Examples, an hPL was clarified before an experiment was performed. Specifically, an hPL in the form of a frozen product was melted at room temperature, then centrifuged at 2000 ×g for 5 minutes to obtain a supernatant, which was used as an hPL within two days. Trypsin (at a final concentration of 83.33 µg/mL) (Roche, Lot No.14528000 (the specific activity: 235 U/mg, the activity (Activity (AZ); with chromozyme): 17,131 U/mL)) with EDTA (at a final concentration of 1 mM) was prepared by dilution with an HBSS (-) to obtain a diluted trypsin liquid. To 500 µL of the diluted trypsin liquid, a trypsin inhibitor candidate liquid (a HSA (Albuminar 25% I.V. injection (CSL Behring K.K.)), an hPL) was added in the range of from 0 to 200 µL, and the trypsin activity was measured using a trypsin activity measurement kit: Amplite Universal Fluorimetric Protease Activity assay Kit (model number 13500, AAT Bioquest, Inc.). Assuming that the trypsin activity of the diluted trypsin liquid with no hPL added thereto was 1, the trypsin activity of each sample was corrected in accordance with (the measurement value of the trypsin activity of each sample) × (the amount of the diluted trypsin liquid + the amount of the trypsin inhibitor candidate liquid)/(the amount of the diluted trypsin liquid)/(the trypsin activity of the diluted trypsin liquid). The results of evaluation of the activity of inhibiting trypsin by an HSA and an hPL are shown in Figure 2. The trypsin activity was rapidly decreased (the trypsin activity was inhibited irreversibly) at 1.44 µL or more as the amount of an hPL which is contacted per µg of trypsin, but no rapid decrease in the activity was observed with an HSA, showing that an hPL was better as a trypsin inhibitor. The results revealed that, under the condition of contacting 2.4 µL or more of an hPL which is contacted per µg of trypsin, the trypsin activity was inhibited almost completely.

### (Example 3) Examination of the concentration of trypsin for releasing

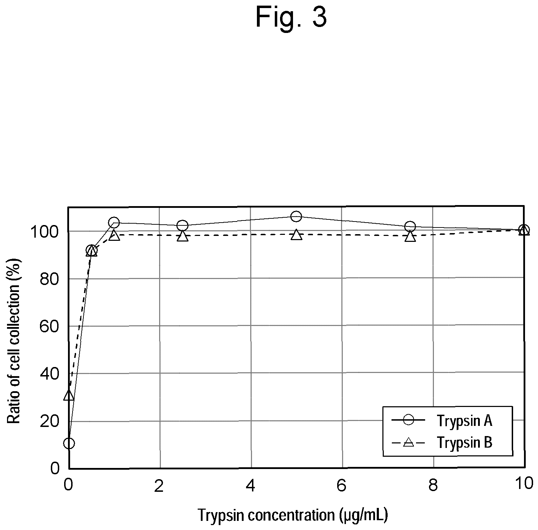

Mesenchymal cells were cultured in a T75 flask by the method described in WO2020/251020. Trypsin was added to an HBSS (-) containing EDTA (a final concentration of 1 mM), and diluted trypsin liquids (trypsin A: Lot No.45022200 (the specific activity: 234 U/mg, the activity: 17,131 U/mL) and trypsin B: Lot No.48904000 (the specific activity: 256 U/mg, the activity: 17,438 U/mL)) were prepared having a trypsin concentration of 0.5, 1.0, 2.5, 5.0, 7.5, and 10.0 µg/mL respectively. The culture medium was removed from the T75 flask, and washed with an HBSS (-). Diluted trypsin liquid in an amount of 2 mL or an HBSS (-) containing EDTA (a final concentration of 1 mM) as a blank was added respectively, and the resulting mixture was left to stand at 20°C for 10 minutes to release the cells. All of the released cells were collected, and the number of the cells was counted using a cell counter NC-200 (M&S TechnoSystems, Inc.). Assuming that the number of cells collected when treated at a trypsin concentration of 10.0 of µg/mL was 100%, the ratio of the cells collected at each trypsin concentration was calculated (Figure 3). At a trypsin concentration of 1.0 µg/mL or more, the cells were collected without loss.

### (Example 4) Examination of the amount of an hPL to be added when trypsin concentration is low

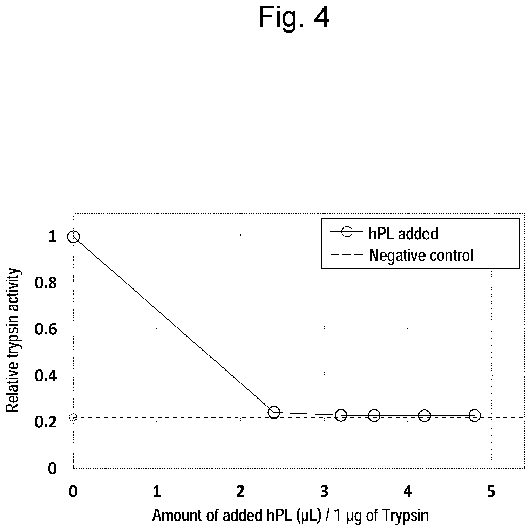

Trypsin (a final concentration of 3.0 µg/mL) (Lot No. 48904000) with EDTA (a final concentration of 1 mM) was prepared by dilution with an HBSS (-) to obtain a diluted trypsin liquid. To 5 mL of the diluted trypsin liquid, an hPL was added in the range of from 0 to 72 µL in such a manner that the amount of an hPL added (µL) / 1 µg trypsin concentration was 0, 2.4, 3.2, 3.6, 4.2, or 4.8, respectively. The trypsin activity was measured using the trypsin activity measurement kit described in Example 2. Assuming that the trypsin activity of the diluted trypsin liquid (with no hPL added thereto) was 1, the results of evaluation of the activity of inhibiting trypsin with respect to the addition of an hPL are shown in Figure 4. The negative control (a value of activity without trypsin) is drawn with a dotted line (approximately 0.22). The results revealed that, when the amount of hPL to be contacted was 2.4 µL or more per µg of trypsin, the trypsin activity was inhibited almost completely. This result revealed that, when the total amount of protein in the hPL to be contacted was 132 µg or more per µg of trypsin, the trypsin activity was inhibited almost completely.

All the publications, patents, and patent applications cited herein are incorporated herein by reference in their entirety.

## Claims

1. A method for producing a cell preparation, comprising:
a step of culturing a cell which adheres to a base material,
a step of releasing the cell from the base material using trypsin, and
a step of contacting a human platelet lysate (hPL) with both the trypsin and the cell after said releasing.

2. The method for producing a cell preparation according to claim 1, wherein the hPL is clarified.

3. The method for producing a cell preparation according to claim 1 or 2, wherein the hPL is not diluted.

4. The method for producing a cell preparation according to any one of claims 1 to 3, wherein the amount of the contacted hPL is 1.44 µL or more per µg of the trypsin.

5. The method for producing a cell preparation according to claim 4, wherein the amount of the contacted hPL is 2.4 µL or more per µg of the trypsin.

6. The method for producing a cell preparation according to claim 5, wherein the cell is treated with trypsin in the concentration of is 0.9 µg/mL or more and 83.3 µg/mL or less in the step of releasing the cell from the base material using trypsin.

7. The method for producing a cell preparation according to claim 6, wherein the trypsin treatment is performed at 10°C or higher and 37°C or lower in the step of releasing the cell from the base material using trypsin.

8. The method for producing a cell preparation according to any one of claims 1 to 7, wherein the cell is cultured using a culture liquid comprising an hPL in the step of culturing a cell which adheres to a base material.

9. The method for producing a cell preparation according to any one of claims 1 to 8, wherein the cell expresses a platelet-derived growth factor receptor (PDGFR).

10. The method for producing a cell preparation according to any one of claims 1 to 9, wherein the cell is a mesenchymal stem cell (MSC).

11. The method for producing a cell preparation according to claim 10, wherein the MSC is derived from an amnion.

12. A cell preparation produced using a method for producing a cell preparation, the method comprising:
a step of culturing a cell which adheres to a base material,
a step of releasing the cell from the base material using trypsin, and
a step of contacting 1.44 µL or more of an hPL per µg of the trypsin with both the trypsin and the cell after said releasing.
